# EUROPEAN PATENT APPLICATION

(11) **EP 4 715 288 A1**
(43) Date of publication of application: **25.03.2026**
(21) Application number: 24876109.0
(22) Date of filing: 25.06.2024
(51) Int. Cl.: F25D 15/00, F25D 23/00, F25D 25/04, F25D 29/00, F25D 11/00, B65D 81/18, B65G 1/04

(54) **LOW-TEMPERATURE STORAGE SYSTEM FOR SAMPLES**

(30) Priority: 09.10.2023 CN 202311299107
(71) Applicant: SHANGHAI ORIGINCELL BIOLOGICAL CRYO EQUIPMENT CO., LTD., Shanghai 201203 (CN)
(72) Inventor: QU, Jianguo, Shanghai 201203 (CN); XIE, Zhaogao, Shanghai 201203 (CN); XUE, Yunfeng, Shanghai 201203 (CN); NI, Shouchun, Shanghai 201203 (CN)
(74) Representative: Bayramoglu et al.
(86) International application number: PCT/CN2024/101290
(87) International publication number: WO 2025/077286

(57) **Abstract**

The present invention discloses a sample cryogenic storage system, including an operation chamber and a storage chamber, where the operation chamber is disposed above the storage chamber and may perform an access operation on a sample stored in the storage chamber; a plurality of storage ports are disposed in an upper end surface of the storage chamber; and a lifting well assembly is disposed in the operation chamber, is movably disposed in the operation chamber, and is movable to the corresponding storage ports to lift a lifting basket in the storage chamber. The present invention has the following beneficial effects: the lifting well assembly lifts the lifting basket in the operation chamber, a shovel plate assembly may push out a plate rack of the lifting basket, a rotary gripping assembly may rotate at 360 degrees to grip the plate rack or the sample, a jacking-up mechanism may jack up a transfer tank and dock the transfer tank with the operation chamber, and the rotary gripping assembly may store the sample or the plate rack from the transfer tank into the lifting basket through the shovel plate assembly and the lifting well assembly, so as to store the lifting basket in the storage chamber, thereby greatly improving the work efficiency.

## Description

### TECHNICAL FIELD

The present invention relates to the technical field of sample storage, and in particular to, a sample cryogenic storage system.

### BACKGROUND

In the biomedical field, sample cryopreservation equipment is used for storing biological samples such as blood samples, vaccines and bacterial and viral strains, such that the samples are continuously maintained in liquid nitrogen for long-term preservation of activity. A plurality of sample boxes are stored in the equipment, a plurality of test tubes are arranged in the sample boxes, and samples required to be stored are in the test tubes.

In current cryopreservation equipment, in the process of extracting biological samples, a lifting well mechanism needs to be additionally disposed above storage equipment to extract a lifting basket from the storage equipment, and then an external fixed shovel plate mechanism exports the sample boxes in the lifting basket, such that a large space is occupied, and the fixed lifting well mechanism is less convenient. It is inconvenient to extract the lifting basket for a refrigerator with a large storage range, such that the work efficiency is reduced. Therefore, there is an urgent need for a sample access system.

### SUMMARY

The objective of this section is to summarize some aspects of the embodiments of the present invention and briefly introduce some preferred embodiments. In this section and the Abstract and invention name of the present application, some simplifications or omissions may be made to avoid the objectives of this section, the Abstract and invention name to become fuzzy, and such the simplifications or omissions cannot be used for limiting the scope of the present invention.

In view of the problems as described above or existing in the prior art, the present invention is proposed.

Therefore, an objective of the present invention is to provide a sample cryogenic storage system. A lifting well assembly moves in a storage chamber and lifts to extract the lifting basket in the storage chamber upward. A shovel plate assembly may shovel a sample plate rack on the lifting basket. A rotary gripping assembly may transfer the sample plate rack and may further pick a sample tube on the plate rack. A jacking-up mechanism may dock a transfer tank with an operation chamber, and the rotary gripping assembly may open an upper cover of the transfer tank and place the upper cover aside. A cover opening assembly may open an insulating cover on the storage chamber.

To solve the above technical problems, the present invention provides the following technical solution: A sample cryogenic storage system, including an operation chamber and a storage chamber, where the operation chamber is disposed above the storage chamber and may perform access operations on a sample stored in the storage chamber;
a plurality of storage ports are disposed in an upper end surface of the storage chamber; and
a lifting well assembly is disposed in the operation chamber, is movably disposed in the operation chamber, and is movable to the corresponding storage ports to lift a lifting basket in the storage chamber.

As a preferred solution of the sample cryogenic storage system disclosed by the present invention, a rotary gripping assembly is further disposed in the operation chamber, and the rotary gripping assembly is capable of lifting and rotating to grip a biological plate rack or biological sample.

As a preferred solution of the sample cryogenic storage system disclosed by the present invention, a shovel plate assembly is disposed at a periphery of the lifting well assembly, a plate rack access channel is disposed on the lifting well assembly, and the shovel plate assembly is capable of shoveling or storing a plate rack into/from the lifting basket through the plate rack access channel.

As a preferred solution of the sample cryogenic storage system disclosed by the present invention, a cover opening assembly is further disposed in the operation chamber, and the cover opening assembly is capable of lifting and gripping insulating covers on the storage ports.

As a preferred solution of the sample cryogenic storage system disclosed by the present invention, the lifting well assembly includes a supporting moving member, a supporting shell sliding member, and a lifting hooking member;
the supporting moving member is slidably connected to the operation chamber, the supporting shell sliding member is vertically or transversely slidably connected to the supporting moving member, the lifting hooking member is disposed in the supporting shell sliding member, and the lifting hooking member is capable of lifting the lifting basket in the storage chamber through the storage ports.

As a preferred solution of the sample cryogenic storage system disclosed by the present invention, the supporting shell sliding member includes a supporting shell, a transverse sliding frame of the supporting shell, a vertical sliding supporting frame of the supporting shell, a vertical guide rail of the supporting shell, and a vertical sliding block;
bottom sides of both ends of the transverse sliding frame of the supporting shell are transversely slidably connected to the first guide rail of the supporting shell and the second guide rail of the supporting shell, one side of the transverse sliding frame of the supporting shell is connected to the vertical sliding supporting frame of the supporting shell, the vertical guide rail of the supporting shell and a first driving mechanism are disposed in the vertical sliding supporting frame of the supporting shell, a vertical straight slotted hole is formed in a side surface of the vertical sliding supporting frame of the supporting shell, the vertical sliding block is slidably connected to the vertical guide rail of the supporting shell, the vertical sliding block passes through the vertical straight slotted hole through a connecting shaft and is connected to the supporting shell, the supporting shell is disposed between a first guide rail of the supporting shell and a second guide rail of the supporting shell, a vertical guide rail shaft is disposed on the side surface of the supporting shell, a sliding block of the vertical guide rail shaft is disposed on a side surface of the vertical sliding supporting frame of the supporting shell, and the vertical guide rail shaft is vertically slidably connected to the sliding block of the vertical guide rail shaft.

As a preferred solution of the sample cryogenic storage system disclosed by the present invention, the lifting hooking member includes a handle sliding block, a lifting portion, a handle sliding block guide rail, an arc-shaped baffle block, and a rotary lifting member;
the handle sliding block guide rail is disposed on an inner wall of the supporting shell, the handle sliding block is vertically slidably connected to the handle sliding block guide rail, a lower end surface of the handle sliding block is connected to the lifting portion, a second driving mechanism is disposed on the supporting shell, the second driving mechanism is capable of driving the rotary lifting member to rotate, the rotary lifting member is connected to the handle sliding block, the rotary lifting member is capable of driving the handle sliding block to lift along the handle sliding block guide rail, and the arc-shaped baffle block is disposed on an upper side of the inner wall of the supporting shell.

As a preferred solution of the sample cryogenic storage system disclosed by the present invention, the rotary gripping assembly includes a rotary jaw member, a straw gripping member, and a gripping supporting shaft;
both ends of the gripping supporting shaft are slidably connected to an inner side of the operation chamber, and the straw gripping member and the rotary jaw member are slidably connected to both sides of the gripping supporting shaft, respectively.

As a preferred solution of the sample cryogenic storage system disclosed by the present invention, the rotary jaw member includes a jaw supporting transverse sliding plate, a vertical stroke plate, a rotary shaft driving member, a rotary shaft, a jaw rack, a jaw, and a jaw driving mechanism;
the jaw supporting transverse sliding plate is slidably connected to a side surface of the gripping supporting shaft, the vertical stroke plate is vertically slidably connected to a side surface of the jaw supporting transverse sliding plate, a lower end of the vertical stroke plate is connected to the rotary shaft driving member, the rotary shaft driving member is connected to one end of the rotary shaft, the other end of the rotary shaft is connected to the jaw rack, the jaw driving mechanism is disposed in the jaw rack, and the jaw driving mechanism drives the jaw to grip the biological plate rack.

As a preferred solution of the sample cryogenic storage system disclosed by the present invention, the straw gripping member includes a straw supporting shaft, a straw vertical sliding plate, a straw supporting block, a straw, and a liquid nitrogen cylinder;
the straw supporting shaft is slidably connected to another side surface of the gripping supporting shaft, the straw vertical sliding plate is vertically slidably connected to the straw supporting shaft, the straw supporting block is connected to the straw vertical sliding plate, the straw supporting block is connected to the straw, the liquid nitrogen cylinder is disposed at a periphery of the straw, and the straw is capable of sucking a cryogenic vial.

As a preferred solution of the sample cryogenic storage system disclosed by the present invention, the shovel plate assembly includes a shoveling member and a storage member;
the shoveling member is disposed on the storage member and is capable of jacking up the biological plate rack in the lifting basket through the plate rack access channel, and the storage member is capable of pushing the biological plate rack into the lifting basket through the plate rack access channel.

As a preferred solution of the sample cryogenic storage system disclosed by the present invention, the storage member includes a shovel plate, a sliding push plate, a push block, a sliding push plate supporting plate, a straight tooth plate, a straight tooth plate meshing gear, a gear driving mechanism, and a supporting shell;
the shovel plate is disposed at an inner lower end of the plate rack access channel and is connected to the supporting shell, an outer side of the supporting shell is connected to the sliding push plate supporting plate, the sliding push plate is slidably connected to the sliding push plate supporting plate, the sliding push plate is sleeved at a periphery of the supporting shell, the sliding push plate is connected to the push block, one side of the sliding push plate is connected to the straight tooth plate, the gear driving mechanism is connected to the straight tooth plate meshing gear, the straight tooth plate meshing gear is in meshing connection to the straight tooth plate, and the sliding push plate supporting plate is connected to the gear driving mechanism.

As a preferred solution of the sample cryogenic storage system disclosed by the present invention, the shoveling member includes a push rod, a push rod straight tooth plate, a push rod straight tooth plate supporting shaft, and a push rod driving mechanism;
the push rod straight tooth plate supporting shaft is connected to the sliding push plate supporting plate, the push rod straight tooth plate supporting shaft is disposed at an end away from the push block, the push rod straight tooth plate is slidably connected to the push rod straight tooth plate supporting shaft, a front end of the push rod straight tooth plate is connected to the push rod, an upper end of the push rod straight tooth plate is in meshing connection to the push rod driving mechanism, and the push rod straight tooth plate meshing gear is capable of driving the push rod driving mechanism.

As a preferred solution of the sample cryogenic storage system disclosed by the present invention, the cover opening assembly includes a cover opening supporting shaft, a driving lifting mechanism, a telescopic rod, a supporting rod, a hooking portion, and a guide bar;
the cover opening supporting shaft is slidably connected to the inner side of the operation chamber, the driving lifting mechanism is disposed on the cover opening supporting shaft, an upper end of the telescopic rod is connected to the driving lifting mechanism, a lower end of the telescopic rod is connected to the supporting rod, both ends of the supporting rod are connected to the hooking portion, and the guide bar is connected to the supporting rod.

As a preferred solution of the sample cryogenic storage system disclosed by the present invention, the sample cryogenic storage system further includes a jacking-up mechanism, wherein the jacking-up mechanism is capable of driving a transfer tank to jack up and dock the transfer tank with the operation chamber, and a transfer tank channel port is disposed in the operation chamber.

As a preferred solution of the sample cryogenic storage system disclosed by the present invention, the jacking-up mechanism includes a seventh driving member, a seventh guide rail, a seventh lifting rack, and a series-connected conveyor belt;
the seventh lifting rack is vertically and slidably connected to the seventh guide rail, the transfer tank is capable of being placed on an upper end surface of the seventh lifting rack, the seventh driving member is capable of driving the seventh lifting rack to lift along the seventh guide rail, and the series-connected conveyor belt is disposed on an upper end surface of the seventh guide rail.

As a preferred solution of the sample cryogenic storage system disclosed by the present invention, the operation chamber further includes an incoming identification member and a rotary cleaning member, the incoming identification member is capable of identifying a barcode of the sample or the plate rack, and the rotary cleaning member is capable of cleaning the sample or the plate rack.

The present invention has the following beneficial effects: 1, in the present invention, the samples in the lifting baskets may be stored through the storage chamber, and the storage chamber may store a plurality of groups of lifting baskets; 2, the lifting well assembly moves and lifts in the operation chamber to lift the target lifting basket, and by disposing the movable lifting well assembly, the lifting basket in the large-capacity storage chamber is conveniently lifted, such that a lot of space is saved; 3, the lifting well assembly lifts the lifting basket, such that the plate rack or storage groove in each layer of the lifting basket is located at the position of the plate rack access channel; 4, the shovel plate assembly may shovel or store the plate rack in each layer of the lifting basket; 5, the rotary gripping assembly rotates at 360 degrees may shovel or store the biological plate rack on the lifting basket in the operation chamber; 6, the gripper on the rotary gripping assembly rotates to grip the plate rack or sample tube in the operation chamber for rotary scanning and cleaning; 7, the cover opening assembly may lift and move the insulating cover on the storage chamber, and the insulating cover may cover the storage port to insulate the storage chamber; and 8, the jacking-up mechanism may jack up the transfer tank and dock the transfer tank with the operation chamber, and the rotary gripping assembly may store the sample or plate rack in the transfer tank in the lifting basket through the shovel plate assembly and the lifting well assembly, and the lifting basket is stored in the storage chamber, such that the work efficiency is greatly improved.

### BRIEF DESCRIPTION OF THE DRAWINGS

To more clearly explain the technical solutions of the embodiments of the present invention, drawings required to be used in the descriptions of the embodiments will be simply introduced, obviously, the drawings in the following description are merely some embodiments of the present invention, persons of ordinary skill in the art can also obtain other drawings according to these drawings without creative efforts. In the figures:
FIG. 1 is an overall schematic view of a sample cryogenic storage system.
FIG. 2 is a schematic view of an operation chamber of a sample cryogenic storage system.
FIG. 3 is a sectional view of a storage chamber of a sample cryogenic storage system.
FIG. 4 is a perspective view of a storage chamber of a sample cryogenic storage system.
FIG. 5 is a schematic view of a cover opening assembly of a sample cryogenic storage system.
FIG. 6 is a schematic view of a rotary gripping assembly and a lifting well assembly of a sample cryogenic storage system.
FIG. 7 is a perspective schematic view of a rotary gripping assembly of a sample cryogenic storage system.
FIG. 8 is a schematic view of a supporting shell sliding member of a sample cryogenic storage system.
FIG. 9 is a schematic view of a shovel plate assembly of a sample cryogenic storage system.
FIG. 10 is a partial sectional view of a supporting shell sliding member of a sample cryogenic storage system.
FIG. 11 is a schematic view of a shovel plate assembly of a sample cryogenic storage system.
FIG. 12 is a schematic view of a lifting hooking member of a sample cryogenic storage system.
FIG. 13 is a partially enlarged view of a lifting hooking member of a sample cryogenic storage system.
FIG. 14 is a schematic view of a rotary gripping assembly of a sample cryogenic storage system.
FIG. 15 is a schematic view of a jacking-up assembly of a sample cryogenic storage system.
FIG. 16 is a schematic view of a jacking-up assembly of a sample cryogenic storage system.
FIG. 17 is a schematic view of a rotary cleaning member of a sample cryogenic storage system.

### Reference numerals:

1, operation chamber;
2, storage chamber;
3, lifting well assembly;
4, rotary gripping assembly;
5, shovel plate assembly;
6, cover opening assembly;
31, supporting moving member;
32, supporting shell sliding member;
33, lifting hooking member;
3201, supporting shell;
3202, transverse sliding frame of supporting shell;
3203, vertical sliding supporting frame of supporting shell;
3204, vertical guide rail of supporting shell;
3205, vertical sliding block;
3301, handle sliding block;
3302, lifting portion;
3303, handle sliding block guide rail;
3305, arc-shaped baffle block;
3304, rotary lifting member;
41, rotary jaw member;
42, straw gripping member;
43, gripping supporting shaft;
4101, jaw supporting transverse sliding plate;
4102, vertical stroke plate;
4103, rotary shaft driving member;
4104, rotary shaft;
4105, jaw rack;
4106, jaw;
4107, jaw driving mechanism;
4201, straw supporting shaft;
4202, straw vertical sliding plate;
4203, straw supporting block;
4204, straw;
4205, liquid nitrogen cylinder;
51, shoveling member;
52, storage member;
5201, shovel plate;
5202, sliding push plate;
5203, push block;
5204, sliding push plate supporting plate;
5205, straight tooth plate;
5206, straight tooth plate meshing gear;
5207, gear driving mechanism;
5101, push rod;
5102, push rod straight tooth plate;
5103, push rod straight tooth plate supporting shaft;
5104, push rod driving mechanism;
601, cover opening supporting shaft;
602, driving lifting mechanism;
603, telescopic rod;
604, supporting rod;
605, hooking portion;
606, guide bar;
7, jacking-up mechanism;
701, seventh driving member;
702, seventh guide rail;
703, seventh lifting rack;
704, series-connected conveyor belt;
8, incoming identification member;
9, rotary cleaning member.

### DETAILED DESCRIPTION OF THE EMBODIMENTS

To make the above objective, features and advantages of the present invention more obvious and understandable, the specific embodiments of the present invention will be described in detail with reference to the drawings.

Many specific details will be set forth in the following description so as to sufficiently understand the present invention, however, the present invention can also be implemented by using other methods different from those described herein, those skilled in the art can make similar promotion without prejudice to the content of the present invention, and therefore the present invention is not limited by specific embodiments disclosed below.

Next, "one embodiment" or "embodiment" herein refers to specific features, structures or characteristics contained in at least one implementation mode of the present invention. "In one embodiment" occurring in different places of this specification does not refer to the same embodiment, nor are separate or selective embodiments mutually exclusive of other embodiments.

### Example 1

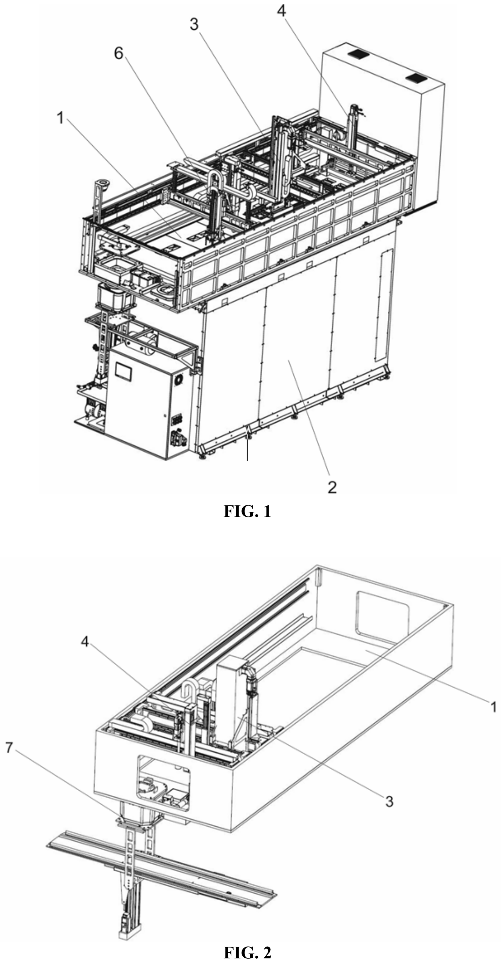

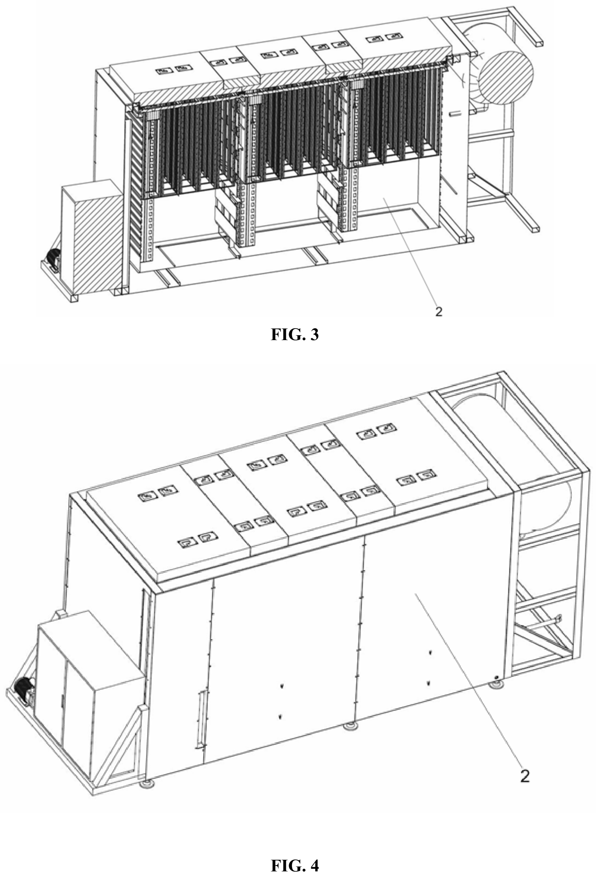

Referring to FIGS. 1-4, as a first embodiment of the present invention, this embodiment provides a sample cryogenic storage system, including an operation chamber 1 and a storage chamber 2, where the storage chamber 2 may store a plurality of groups of lifting baskets, a plurality of biological plate racks may be stored in the lifting baskets, biological samples are placed in the plate rack, and the lifting well assembly 3 may move and lift in the operation chamber 1 to lift the lifting baskets in the storage chamber 2 in the operation chamber 1.

Specifically, the sample cryogenic storage system includes the operation chamber 1 and the storage chamber 2, where the operation chamber 1 is disposed above the storage chamber 2 and may perform an access operation on a sample in the storage chamber 2;
a plurality of storage ports are disposed on an upper end surface of the storage chamber 2; and
a lifting well assembly 3 is disposed in the operation chamber 1, is movably disposed in the operation chamber 1, and is movable to the corresponding storage ports to lift the lifting basket in the storage chamber 2.

To sum up, the plurality of groups of lifting baskets are stored in the storage chamber 2, the plurality of biological plate racks may be stored in the lifting baskets, a plurality of sample tubes may be placed in the biological plate racks, the lifting well assembly 3 moves and lifts in the operation chamber 1 to lift the target lifting basket to the storage chamber 2, and the target lifting basket passes through the access plate rack and is placed back in the storage chamber 2 for freezing. By disposing the movable lifting well assembly 3, the lifting basket in the large-capacity storage chamber 2 is conveniently lifted, such that a lot of space is saved.

### Example 2

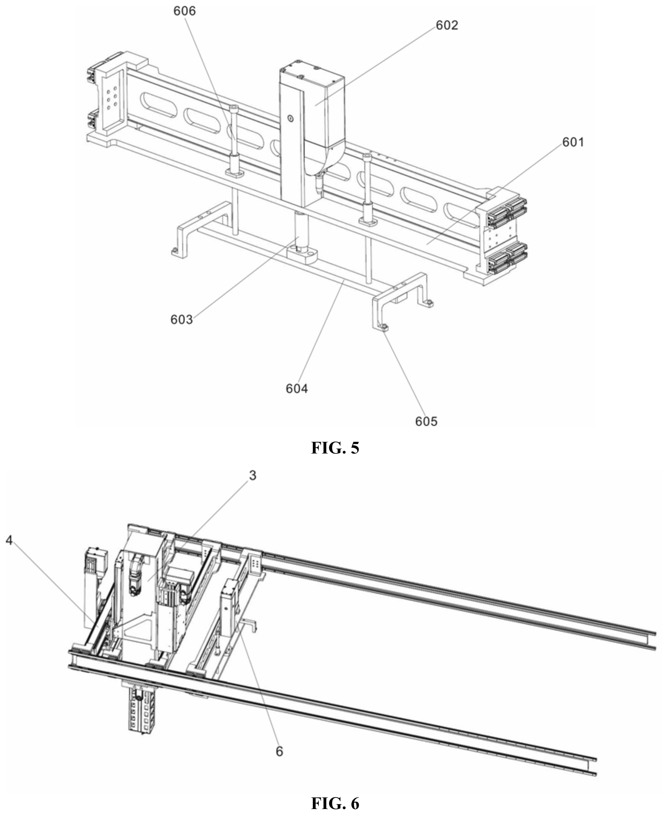

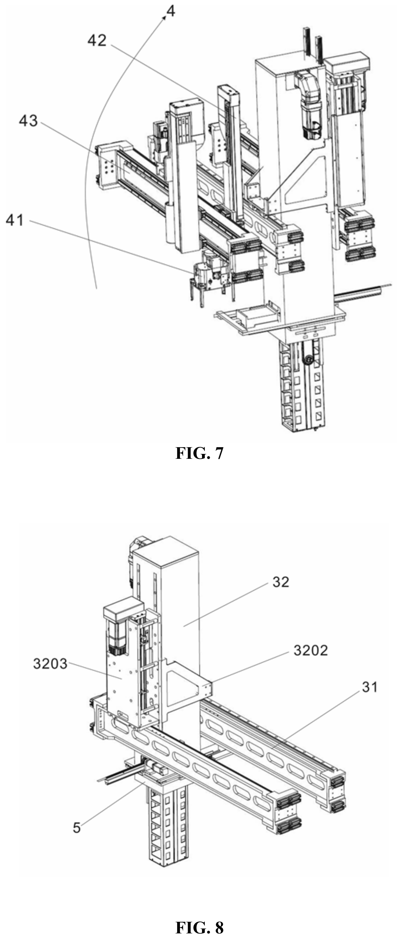

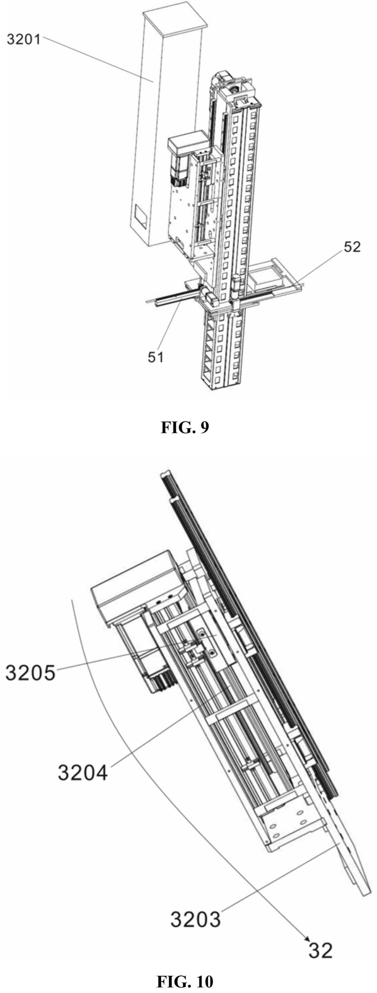

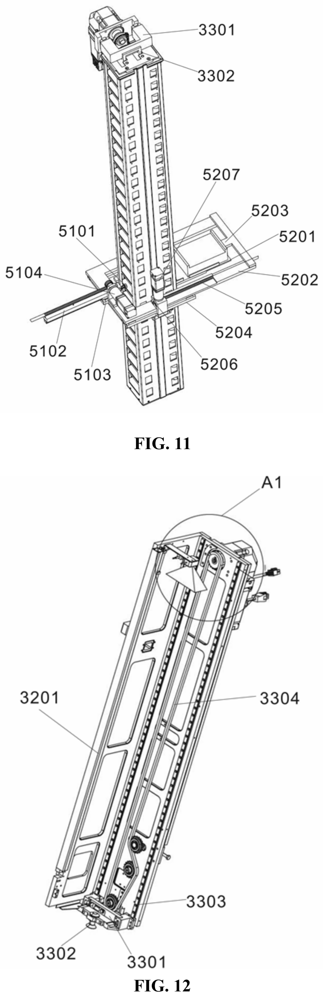

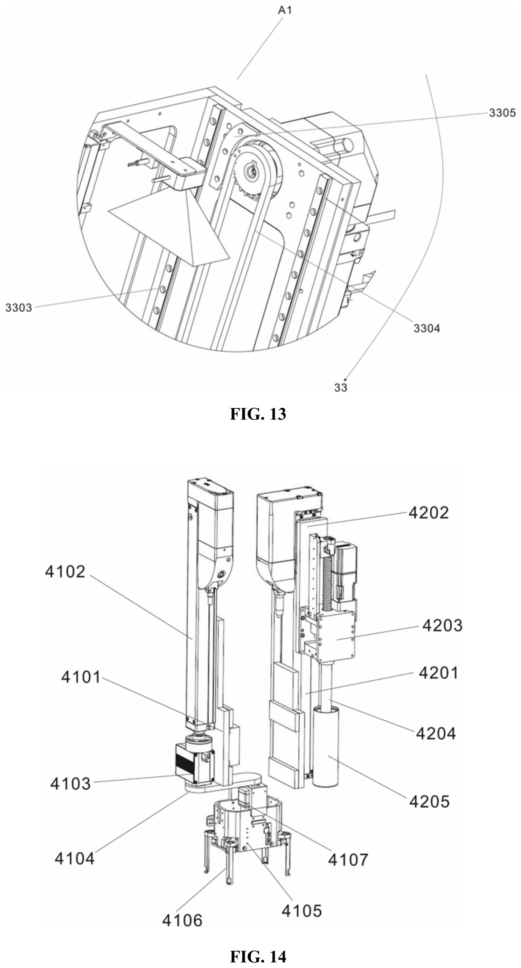

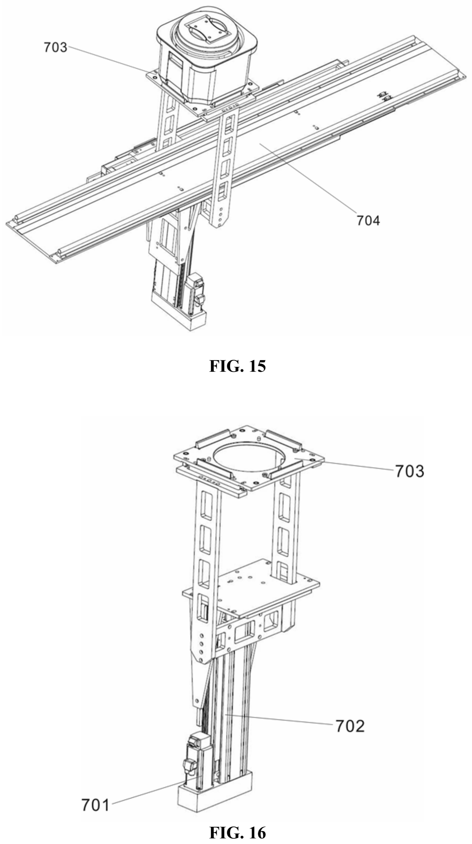

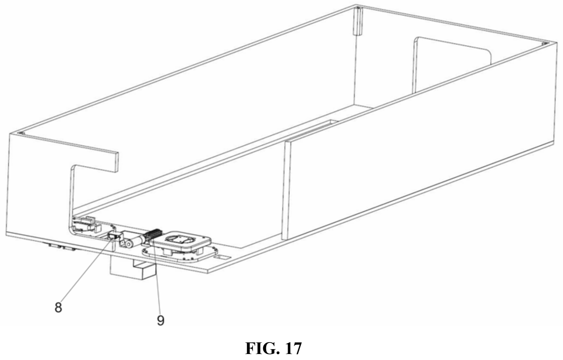

Referring to FIGS. 1-17, as a second embodiment of the present invention, in the previous embodiment, the sample cryogenic storage system includes an operation chamber 1 and a storage chamber 2, where the storage chamber 2 may store a plurality of groups of lifting baskets, a plurality of biological plate racks may be stored in the lifting baskets, biological samples are placed in the plate rack, and the lifting well assembly 3 may move and lift in the operation chamber 1 to lift the lifting baskets in the storage chamber 2 in the operation chamber 1.

Specifically, the sample cryogenic storage system includes the operation chamber 1 and the storage chamber 2, where the operation chamber 1 is disposed above the storage chamber 2 and may perform an access operation on a sample in the storage chamber 2;
a plurality of storage ports are disposed on an upper end surface of the storage chamber 2; and
a lifting well assembly 3 is disposed in the operation chamber 1, is movably disposed in the operation chamber 1, and is movable to the corresponding storage ports to lift the lifting basket in the storage chamber 2.

Further, a rotary gripping assembly 4 is further disposed in the operation chamber 1, and the rotary gripping assembly 4 is capable of lifting and rotating to grip a biological plate rack or biological sample.

Preferably, the rotary gripping assembly 4 may move in an XYZ axis direction in the operation chamber 1, and the rotary jaw member 41 of the rotary gripping assembly 4 may rotate at any horizontal angle.

Further, a shovel plate assembly 5 is disposed at a periphery of the lifting well assembly 3, a plate rack access channel is disposed on the lifting well assembly 3, and the shovel plate assembly 5 is capable of shoveling or storing a plate rack into/from the lifting basket through the plate rack access channel.

Further, a cover opening assembly 6 is further disposed in the operation chamber 1, and the cover opening assembly 6 is capable of lifting and gripping insulating covers on the storage ports.

Preferably, the cover opening assembly 6 may move in the operation chamber 1 and cover the insulating cover on the corresponding storage chamber 2. Insulating covers are disposed at the plurality of storage ports, and the cover opening assembly 6 may open the insulating covers.

Further, the lifting well assembly 3 includes a supporting moving member 31, a supporting shell sliding member 32, and a lifting hooking member 33;
the supporting moving member 31 is slidably connected to the operation chamber 1, the supporting shell sliding member 32 is in vertical or transverse sliding connection to the supporting moving member 31, the lifting hooking member 33 is disposed in the supporting shell sliding member 32, and the lifting hooking member 33 is capable of lifting the lifting basket in the storage chamber 2 through the storage ports.

Preferably, the supporting moving member 31 is slidably connected along the inner wall of the operation chamber 1, the supporting shell sliding member 32 may move transversely or vertically along the supporting moving member 31, the lifting hooking member 33 is disposed on the inner side of the supporting shell sliding member 32, and the lifting hooking member 33 may lift and slide along the inner side of the supporting shell sliding member 32.

Further, the supporting shell sliding member 32 includes a supporting shell 3201, a transverse sliding frame 3202 of the supporting shell, a vertical sliding supporting frame 3203 of the supporting shell, a vertical guide rail 3204 of the supporting shell, and a vertical sliding block 3205;
bottom sides of both ends of the transverse sliding frame 3202 are transversely slidably connected to a first guide rail 3101 of the supporting shell and a second guide rail 3102 of the supporting shell, one side of the transverse sliding frame 3202 of the supporting shell is connected to the vertical sliding supporting frame 3203 of the supporting shell, the vertical guide rail 3204 of the supporting shell and a first driving mechanism are disposed in the vertical sliding supporting frame 3203 of the supporting shell, a vertical straight slotted hole is formed in a side surface of the vertical sliding supporting frame 3203 of the supporting shell, the vertical sliding block 3205 is slidably connected to the vertical guide rail 3204 of the supporting shell, the vertical sliding block 3205 passes through the vertical straight slotted hole through a connecting shaft and is connected to the supporting shell 3201, the supporting shell 3201 is disposed between the first guide rail 3101 of the supporting shell and the second guide rail 3102 of the supporting shell, a vertical guide rail shaft is disposed on the side surface of the supporting shell 3201, a sliding block of the vertical guide rail shaft is disposed on a side surface of the vertical sliding supporting frame 3203 of the supporting shell, and the vertical guide rail shaft is vertically slidably connected to the sliding block of the vertical guide rail shaft.

Preferably, two groups of supporting moving members 31 may be disposed, guide rails are disposed on the supporting moving members 31, and the supporting shell sliding member 32 may slide thereon.

Preferably, the supporting moving members 31 at the bottom ends of both sides of the transverse sliding frame 3202 of the supporting shell are slidably connected, and a side surface of the transverse sliding frame 3202 of the supporting shell is connected to the vertical sliding supporting frame 3203 of the supporting shell.

Preferably, driven by the first driving mechanism, the vertical sliding block 3205 may be slidably connected along the vertical guide rail 3204 of the supporting shell, the vertical sliding block 3205 passes through the vertical straight slotted hole and is fixedly connected to the supporting shell 3201, and the vertical guide rail shaft of the supporting shell 3201 may be slidably connected up and down along the vertical guide rail shaft when lifting.

Preferably, the rotary shaft driving member 4103, the first driving mechanism, the second driving mechanism, the jaw driving mechanism 4107, the gear driving mechanism 5207, and the push rod driving mechanism 5104 may be driven by a motor, or an air cylinder, or an electric cylinder, or may be hydraulically driven or driven by other means.

Further, a lifting hooking member 33 includes a handle sliding block 3301, a lifting portion 3302, a handle sliding block guide rail 3303, an arc-shaped baffle block 3305, and a rotary lifting member 3304;
the handle sliding block guide rail 3303 is disposed on an inner wall of the supporting shell 3201, the handle sliding block 3301 is vertically slidably connected to the handle sliding block guide rail 3303, a lower end surface of the handle sliding block 3301 is connected to the lifting portion 3302, a second driving mechanism is disposed on the supporting shell 3201, the second driving mechanism is capable of driving the rotary lifting member 3304 to rotate, the rotary lifting member 3304 is connected to the handle sliding block 3301, the rotary lifting member 3304 is capable of driving the handle sliding block 3301 to lift along the handle sliding block guide rail 3303, and the arc-shaped baffle block 3305 is disposed on an upper side of the inner wall of the supporting shell 3201.

Preferably, the rotary lifting member 3304 includes a rotating belt and a rotating wheel, in which the second driving mechanism drives the rotating wheel to rotate, the rotating wheel drives the rotating belt to rotate, one side of the rotating belt is connected to the handle sliding block 3301, and when rotating, the rotating belt may drive the handle sliding block 3301 to lift along the handle sliding block guide rail 3303.

It should be noted that the rotary lifting member 3304 may also be configured as a combination of a sprocket and a chain; the rotary lifting member 3304 may also be a lifting mechanism which directly drives the handle sliding block 3301 to lift.

It should be noted that the handle sliding block 3301, driven by the motor, may also be directly driven to lift along the handle sliding block guide rail 3303.

Preferably, the second driving mechanism drives the rotary lifting member 3304 to drive the handle sliding block 3301 to lift along the handle sliding block guide rail 3303. The handle sliding block 3301 drives the lifting portion 3302 to lift, and the lifting portion 3302 may hook or grip the upper end of the lifting basket, so as to drive the lifting basket to lift.

It should be noted that the plurality of plate racks may be vertically stored in the lifting basket, a plurality of storage lattices are disposed on the lifting basket, and each of the storage lattices may store the plate rack.

Preferably, the lifting portion 3302 may be a jaw or a claw. A visual sensing device is further disposed at an inner upper end of the supporting shell 3201, and the visual sensing device may be a camera or a sensor, which may detect the position where the lifting basket arrives at. The arc-shaped baffle block 3305 has certain elasticity, which may play a role in buffering the lifting basket.

Preferably, the lifting portion 3302 will lift the lifting basket into the supporting shell 3201.

Further, the rotary gripping assembly 4 includes a rotary jaw member 41, a straw gripping member 42, and a gripping supporting shaft 43;
both ends of the gripping supporting shaft 43 are slidably connected to an inner side of the operation chamber 1, and the straw gripping member 42 and the rotary jaw member 41 are slidably connected to both sides of the gripping supporting shaft 43, respectively.

Further, the rotary jaw member 41 includes a jaw supporting transverse sliding plate 4101, a vertical stroke plate 4102, a rotary shaft driving member 4103, a rotary shaft 4104, a jaw rack 4105, a jaw 4106, and a jaw driving mechanism 4107;
the jaw supporting transverse sliding plate 4101 is slidably connected to a side surface of the gripping supporting shaft 43, the vertical stroke plate 4102 is vertically slidably connected to a side surface of the jaw supporting transverse sliding plate 4101, a lower end of the vertical stroke plate 4102 is connected to the rotary shaft driving member 4103, the rotary shaft driving member 4103 is connected to one end of the rotary shaft 4104, the other end of the rotary shaft 4104 is connected to the jaw rack 4105, the jaw driving mechanism 4107 is disposed in the jaw rack 4105, and the jaw driving mechanism 4107 drives the jaw to grip the biological plate rack 4106.

It should be noted that the jaw supporting transverse sliding plate 4101 is transversely slidably connected to the gripping supporting shaft 43, the vertical stroke plate 4102, driven by the motor, is vertically slidably connected to the other side surface of the jaw supporting transverse sliding plate 4101, a lower end of the vertical stroke plate 4102 is connected to the rotary shaft driving member 4103, the rotary shaft driving member 4103 may drive the rotary shaft 4104 to rotate at 360 degrees, the rotary shaft 4104 may drive the jaw rack 4105 and the jaw 4106 to rotate, and the jaw driving mechanism 4107 drives the jaw 4106 to grip the plate rack.

Further, the straw gripping member 42 includes a straw supporting shaft 4201, a straw vertical sliding plate 4202, a straw supporting block 4203, a straw 4204, and a liquid nitrogen cylinder 4205;
the straw supporting shaft 4201 is slidably connected to another side surface of the gripping supporting shaft 43, the straw vertical sliding plate 4202 is vertically slidably connected to the straw supporting shaft 4201, the straw supporting block 4203 is connected to the straw vertical sliding plate 4202, the straw supporting block 4203 is connected to the straw 4204, the liquid nitrogen cylinder 4205 is disposed at a periphery of the straw 4204, and the straw 4204 is capable of sucking a cryogenic vial.

It should be noted that the straw supporting shaft 4201 may be slidably connected to along the other side surface of the gripping supporting shaft 43, the straw vertical sliding plate 4202 may be vertically slidably connected along the other side surface of the straw supporting shaft 4201, the straw vertical sliding plate 4202 is connected to the straw supporting block 4203, the straw supporting block 4203 drives the straw 4204 to lift, the liquid nitrogen cylinder 4205 is disposed at the periphery of the straw 4204, liquid nitrogen may be placed in the liquid nitrogen cylinder 4205, the lower end of the straw 4204 may suck the cryogenic vial, and a vacuum pump is connected to the straw to provide positive and negative gases to the straw 4204, which facilitates suction of the cryogenic vial.

Further, the shovel plate assembly 5 includes a shoveling member 51 and a storage member 52;
the shoveling member 51 is disposed on the storage member 52 and is capable of jacking up the biological plate rack in the lifting basket through the plate rack access channel, and the storage member 52 is capable of pushing the biological plate rack into the lifting basket through the plate rack access channel.

Further, the storage member 52 includes a shovel plate 5201, a sliding push plate 5202, a push block 5203, a sliding push plate supporting plate 5204, a straight tooth plate 5205, a straight tooth plate meshing gear 5206, a gear driving mechanism 5207, and a supporting shell 3201;
the shovel plate 5201 is disposed at an inner lower end of the plate rack access channel and is connected to the supporting shell 3201, an outer side of the supporting shell 3201 is connected to the sliding push plate supporting plate 5204, the sliding push plate 5202 is slidably connected to the sliding push plate supporting plate 5204, the sliding push plate 5202 is sleeved at a periphery of the supporting shell 3201, the sliding push plate 5202 is connected to the push block 5203, one side of the sliding push plate 5202 is connected to the straight tooth plate 5205, the gear driving mechanism 5207 is connected to the straight tooth plate meshing gear 5206, the straight tooth plate meshing gear 5206 is in meshing connection to the straight tooth plate 5205, and the sliding push plate supporting plate 5204 is connected to the gear driving mechanism 5207.

Preferably, the rotary jaw member 41 may grip the plate rack and place the plate rack on the shovel plate 5201, then the gear driving mechanism 5207 drives the straight tooth plate meshing gear 5206 to rotate, the straight tooth plate meshing gear 5206 meshes with the straight tooth plate 5205 to drive the straight tooth plate 5205 and the sliding push plate 5202 to move, the sliding push plate 5202 will drive the push block 5203 to move, the push block 5203 pushes the plate rack to enter the lifting basket, and after the plate rack is accessed, the push block 5203 escapes.

Further, the shoveling member 51 includes a push rod 5101, a push rod straight tooth plate 5102, a push rod straight tooth plate supporting shaft 5103, and a push rod driving mechanism 5104;
the push rod straight tooth plate supporting shaft 5103 is connected to the sliding push plate supporting plate 5204, the push rod straight tooth plate supporting shaft 5103 is disposed at an end away from the push block 5203, the push rod straight tooth plate 5102 is slidably connected to the push rod straight tooth plate supporting shaft 5103, a front end of the push rod straight tooth plate 5102 is connected to the push rod 5101, an upper end of the push rod straight tooth plate 5102 is in meshing connection to the push rod driving mechanism 5104, and the push rod straight tooth plate meshing gear is capable of driving the push rod driving mechanism 5104.

It should be noted that the push rod driving mechanism 5104 will push the push rod gear to rotate, the push rod gear meshes with the push rod straight tooth plate 5102, to drive the push rod straight tooth plate 5102 to move, the push rod straight tooth plate 5102 slides along the push rod straight tooth plate supporting shaft 5103, the push rod straight tooth plate 5102 will drive the push rod 5101 to move forward, and the push rod 5101 will push the plate rack in the lifting basket out of the lifting basket, such that the plate rack falls onto the shovel plate 5201, which facilitates gripping or picking by the rotary gripping assembly 4.

It should be noted that when the lifting basket is in the supporting shell 3201, the push rod 5101 may shovel the plate rack through the access channel; the push block 5203 may also be stored in the plate rack through the access channel, and the lifting basket may be lifted through the lifting hooking member 33, such that the plate rack or a storage vacancy on the lifting basket may be at the position of the access channel, which facilitates access of the lifting basket in each layer.

Further, the cover opening assembly 6 includes a cover opening supporting shaft 601, a driving lifting mechanism 602, a telescopic rod 603, a supporting rod 604, a hooking portion 605, and a guide bar 606;
the cover opening supporting shaft 601 is slidably connected to the inner side of the operation chamber 1, the driving lifting mechanism 602 is disposed on the cover opening supporting shaft 601, an upper end of the telescopic rod 603 is connected to the driving lifting mechanism 602, a lower end of the telescopic rod 603 is connected to the supporting rod 604, both ends of the supporting rod 604 are connected to the hooking portion 605, and the guide bar 606 is connected to the supporting rod 604.

Preferably, the seventh driving member 701 and the driving lifting mechanism 602 may be a motor, or an air cylinder or another driving lifting mechanism;
preferably, the driving lifting mechanism 602 may drive the telescopic rod 603 to lift, the telescopic rod drives the supporting rod 604 to lift, hooking portions 605 are disposed on both sides of the supporting rod 604, the hooking portions 605 may hook the insulating covers on the upper end surface of the storage chamber 2, and the insulating cover may cover the storage ports.

Further, the sample cryogenic storage system further includes a jacking-up mechanism 7, where the jacking-up mechanism 7 is capable of driving a transfer tank to jack up and dock the transfer tank with the operation chamber 1, and a transfer tank channel port is disposed in the operation chamber 1.

Preferably, a tank cover access mechanism is further disposed in the operation chamber 1, the tank cover access mechanism may support a tank cover of the transfer tank, and the tank cover access mechanism may place tank covers with different sizes.

Further, the jacking-up mechanism 7 includes a seventh driving member 701, a seventh guide rail 702, a seventh lifting rack 703, and a series-connected conveyor belt 704;
the seventh lifting rack 703 is vertically and slidably connected to the seventh guide rail 702, the transfer tank is capable of being placed on an upper end surface of the seventh lifting rack 703, the seventh driving member 701 is capable of driving the seventh lifting rack 703 to lift along the seventh guide rail 702, and the series-connected conveyor belt 704 is disposed on an upper end surface of the seventh guide rail 702.

Preferably, the seventh driving member 701 may drive the seventh lifting rack 703 to lift vertically along the seventh guide rail 702, the transfer tank may be placed at the upper end of the seventh lifting rack 703, and the rotary gripping assembly 4 may move a channel cover on a channel opening of the transfer tank aside, which facilitates connection between the transfer tank and the operation chamber 1.

Preferably, the series-connected conveyor belt 704 may be connected in series to a plurality of groups of storage equipment, and the transfer tank and the plurality of storage equipment or other equipment may be transferred through the series-connected conveyor belt 704, which greatly saves the manpower.

Further, the operation chamber 1 further includes an incoming identification member 8 and a rotary cleaning member 9, the incoming identification member 8 is capable of identifying a barcode of the sample or the plate rack, and the rotary cleaning member 9 is capable of cleaning the sample or the plate rack.

Preferably, the incoming identification member 8 may scan and identify a label of the plate rack or sample tube, and the rotary cleaning member 9 may clean frost on the label of the plate rack or sample tube, to avoid the occurrence of unrecognizable phenomena.

Further, a lifting basket rack is disposed in the storage chamber 2, the lifting basket rack may support the lifting basket, the storage chamber 2 has the function of cryogenic storage, which may guarantee the activity of the samples.

To sum up, in the present invention, the cover opening assembly 6 may open the corresponding insulating covers, and the lifting hooking member 33 grips or hooks the lifting basket in the storage chamber 2, such that the lifting basket is lifted to the position of the plate rack access channel. Then the shoveling member 51 may push out the plate rack of the lifting basket at the position of the plate rack access channel, and the rotary gripping member 4 may grip the plate rack pushed out for a next operation, and the lifting hooking member 33 lifts, such that the plate rack in each layer of the lifting basket may correspond to the position of the plate rack access channel. The plate rack may be placed in the storage groove of the lifting basket through the storage member 52. The rotary gripping assembly 4 may also pick the sample tube on the plate rack. The rotary jaw member 41 may rotate at 360 degrees to open the plate rack or the upper cover of the transfer tank, which avoids a condition that the rotary jaw member 41 is not in contact with the plate rack or the upper cover. The jacking-up mechanism 7 may jack up the transfer tank and dock the transfer tank with the operation chamber 1. The rotary gripping assembly 4 may open the upper cover of the transfer tank, and store the cryogenic vial or plate rack therein into the lifting basket through the shovel plate assembly 5 and the lifting well assembly 3, so as to place the sample in the storage chamber 2 for freezing. Moreover, the cryogenic vial and the plate rack are scanned by the incoming identification member 8 during warehousing and ex-warehousing. Before scanning and identifying, frost on the label of the plate rack or cryogenic vial is cleaned to prevent a condition that scanning cannot be performed. The device achieves an automatic operation and greatly improves the work efficiency.

Importantly, it should be noted that the configurations and arrangements of the present application shown in multiple different exemplary embodiments are only illustrative. Although several embodiments are only described in detail in the disclosure here, persons who refer to the disclosure will readily appreciate that many modifications may be possible (for example, sizes, scales, structures, shapes and proportions of various elements, as well as parameters (for example, temperature, pressure, etc.), installation arrangement, use of materials, color, change in orientation, etc.) without substantially departing the novel teachings and advantages of the subject described in the present application. For example, an element shown as being integrally formed may be composed of multiple parts or elements, the positions of the elements may be reversed or otherwise varied, and the nature or number or position of discrete elements may be altered or varied. Therefore, all of such the modifications are intended to be included within the scope of the present invention. The order or sequence of any processes or method steps is changed or rearranged according to alternative embodiments. In claims, any clause of "device plus function" is intended to cover the structures described herein for implementing the functions, and is not only structurally equivalent but also an equivalent structure. Other replacements, modifications, changes and omissions can be made in the design, running state and arrangement of exemplary embodiments without departing from the scope of the present invention. Therefore, the present invention is not limited to specific embodiments, but extends to various modifications which still fall within the scope of the appended claims.

In addition, all features (i.e., those features irrelevant to the best mode presently contemplated for carrying out the present invention, or those features irrelevant to implementing the present invention) of actual embodiments can be not described in order to provide the brief description of exemplary embodiments.

It should be understood that a large amount of specific embodiments can be made in the development process of any actual embodiments such as any projects or design items. Such development efforts might be complex and time-consuming, but would not require undue experimentation for ordinary technicians having the benefit of this disclosure, which would be a routine work of design, manufacture and production.

It should be noted that the embodiments described above are merely for illustrating, but not limiting, the technical solutions of the present invention, although the present invention has been described in detail with reference to preferred embodiments, persons of ordinary skill in the art should be understood that the technical solutions of the present invention can be amended or equivalently replaced, all of which should be covered within the scope of claims of the present invention without departing from the spirit and scope of the technical solutions of the present invention .

## Claims

1. A sample cryogenic storage system, **characterized by** comprising an operation chamber and a storage chamber, wherein the operation chamber is disposed above the storage chamber and is capable of performing an access operation on a sample stored in the storage chamber;
a plurality of storage ports are disposed in an upper end surface of the storage chamber; and
a lifting well assembly is disposed in the operation chamber, is movably disposed in the operation chamber, and is movable to the corresponding storage ports to lift a lifting basket in the storage chamber.

2. The sample cryogenic storage system according to claim 1, **characterized in that** a rotary gripping assembly is further disposed in the operation chamber, and the rotary gripping assembly is capable of lifting and rotating to grip a biological plate rack or biological sample.

3. The sample cryogenic storage system according to claim 2, **characterized in that** a shovel plate assembly is disposed at a periphery of the lifting well assembly, a plate rack access channel is disposed on the lifting well assembly, and the shovel plate assembly is capable of shoveling or storing a plate rack into/from the lifting basket through the plate rack access channel.

4. The sample cryogenic storage system according to claim 1, **characterized in that** a cover opening assembly is further disposed in the operation chamber, and the cover opening assembly is capable of lifting and gripping insulating covers on the storage ports.

5. The sample cryogenic storage system according to any one of claims 1 to 4, **characterized in that** the lifting well assembly comprises a supporting moving member, a supporting shell sliding member, and a lifting hooking member; and
the supporting moving member is slidably connected to the operation chamber, the supporting shell sliding member is in vertical or transverse sliding connection to the supporting moving member, the lifting hooking member is disposed in the supporting shell sliding member, and the lifting hooking member is capable of lifting the lifting basket in the storage chamber through the storage ports.

6. The sample cryogenic storage system according to claim 5, **characterized in that** the supporting shell sliding member comprises a supporting shell, a transverse sliding frame of the supporting shell, a vertical sliding supporting frame of the supporting shell, a vertical guide rail of the supporting shell, and a vertical sliding block; and
bottom sides of both ends of the transverse sliding frame of the supporting shell are transversely slidably connected to the supporting moving member, one side of the transverse sliding frame of the supporting shell is connected to the vertical sliding supporting frame of the supporting shell, the vertical guide rail of the supporting shell and a first driving mechanism are disposed in the vertical sliding supporting frame of the supporting shell, a vertical straight slotted hole is formed in a side surface of the vertical sliding supporting frame of the supporting shell, the vertical sliding block is slidably connected to the vertical guide rail of the supporting shell, the vertical sliding block passes through the vertical straight slotted hole through a connecting shaft and is connected to the supporting shell, the supporting shell is disposed between a first guide rail of the supporting shell and a second guide rail of the supporting shell, a vertical guide rail shaft is disposed on the side surface of the supporting shell, a sliding block of the vertical guide rail shaft is disposed on a side surface of the vertical sliding supporting frame of the supporting shell, and the vertical guide rail shaft is vertically slidably connected to the sliding block of the vertical guide rail shaft.

7. The sample cryogenic storage system according to claim 6, **characterized in that** the lifting hooking member comprises a handle sliding block, a lifting portion, a handle sliding block guide rail, an arc-shaped baffle block, and a rotary lifting member; and
the handle sliding block guide rail is disposed on an inner wall of the supporting shell, the handle sliding block is vertically slidably connected to the handle sliding block guide rail, a lower end surface of the handle sliding block is connected to the lifting portion, a second driving mechanism is disposed on the supporting shell, the second driving mechanism is capable of driving the rotary lifting member to rotate, the rotary lifting member is connected to the handle sliding block, the rotary lifting member is capable of driving the handle sliding block to lift along the handle sliding block guide rail, and the arc-shaped baffle block is disposed on an upper side of the inner wall of the supporting shell.

8. The sample cryogenic storage system according to claim 2, **characterized in that** the rotary gripping assembly comprises a rotary jaw member, a straw gripping member, and a gripping supporting shaft; and
both ends of the gripping supporting shaft are slidably connected to an inner side of the operation chamber, and the straw gripping member and the rotary jaw member are slidably connected to both sides of the gripping supporting shaft, respectively.

9. The sample cryogenic storage system according to claim 8, **characterized in that** the rotary jaw member comprises a jaw supporting transverse sliding plate, a vertical stroke plate, a rotary shaft driving member, a rotary shaft, a jaw rack, a jaw, and a jaw driving mechanism; and
the jaw supporting transverse sliding plate is slidably connected to a side surface of the gripping supporting shaft, the vertical stroke plate is vertically slidably connected to a side surface of the jaw supporting transverse sliding plate, a lower end of the vertical stroke plate is connected to the rotary shaft driving member, the rotary shaft driving member is connected to one end of the rotary shaft, the other end of the rotary shaft is connected to the jaw rack, the jaw driving mechanism is disposed in the jaw rack, and the jaw driving mechanism drives the jaw to grip the biological plate rack.

10. The sample cryogenic storage system according to claim 8, **characterized in that** the straw gripping member comprises a straw supporting shaft, a straw vertical sliding plate, a straw supporting block, a straw, and a liquid nitrogen cylinder; and
the straw supporting shaft is slidably connected to another side surface of the gripping supporting shaft, the straw vertical sliding plate is vertically slidably connected to the straw supporting shaft, the straw supporting block is connected to the straw vertical sliding plate, the straw supporting block is connected to the straw, the liquid nitrogen cylinder is disposed at a periphery of the straw, and the straw is capable of sucking a cryogenic vial.

11. The sample cryogenic storage system according to claim 3, **characterized in that** the shovel plate assembly comprises a shoveling member and a storage member; and
the shoveling member is disposed on the storage member and is capable of jacking up the biological plate rack in the lifting basket through the plate rack access channel, and the storage member is capable of pushing the biological plate rack into the lifting basket through the plate rack access channel.

12. The sample cryogenic storage system according to claim 11, **characterized in that** the storage member comprises a shovel plate, a sliding push plate, a push block, a sliding push plate supporting plate, a straight tooth plate, a straight tooth plate meshing gear, a gear driving mechanism, and a supporting shell; and
the shovel plate is disposed at an inner lower end of the plate rack access channel and is connected to the supporting shell, an outer side of the supporting shell is connected to the sliding push plate supporting plate, the sliding push plate is slidably connected to the sliding push plate supporting plate, the sliding push plate is sleeved at a periphery of the supporting shell, the sliding push plate is connected to the push block, one side of the sliding push plate is connected to the straight tooth plate, the gear driving mechanism is connected to the straight tooth plate meshing gear, the straight tooth plate meshing gear is in meshing connection to the straight tooth plate, and the sliding push plate supporting plate is connected to the gear driving mechanism.

13. The sample cryogenic storage system according to claim 12, **characterized in that** the shoveling member comprises a push rod, a push rod straight tooth plate, a push rod straight tooth plate supporting shaft, and a push rod driving mechanism; and
the push rod straight tooth plate supporting shaft is connected to the sliding push plate supporting plate, the push rod straight tooth plate supporting shaft is disposed at an end away from the push block, the push rod straight tooth plate is slidably connected to the push rod straight tooth plate supporting shaft, a front end of the push rod straight tooth plate is connected to the push rod, an upper end of the push rod straight tooth plate is in meshing connection to the push rod driving mechanism, and the push rod straight tooth plate meshing gear is capable of driving the push rod driving mechanism.

14. The sample cryogenic storage system according to claim 4, **characterized in that** the cover opening assembly comprises a cover opening supporting shaft, a driving lifting mechanism, a telescopic rod, a supporting rod, a hooking portion, and a guide bar; and
the cover opening supporting shaft is slidably connected to the inner side of the operation chamber, the driving lifting mechanism is disposed on the cover opening supporting shaft, an upper end of the telescopic rod is connected to the driving lifting mechanism, a lower end of the telescopic rod is connected to the supporting rod, both ends of the supporting rod are connected to the hooking portion, and the guide bar is connected to the supporting rod.

15. The sample cryogenic storage system according to claim 1, **characterized by** further comprising a jacking-up mechanism, wherein the jacking-up mechanism is capable of driving a transfer tank to jack up and dock the transfer tank with the operation chamber, and a transfer tank channel port is disposed in the operation chamber.

16. The sample cryogenic storage system according to claim 15, **characterized in that** the jacking-up mechanism comprises a seventh driving member, a seventh guide rail, a seventh lifting rack, and a series-connected conveyor belt; and
the seventh lifting rack is vertically and slidably connected to the seventh guide rail, the transfer tank is capable of being placed on an upper end surface of the seventh lifting rack, the seventh driving member is capable of driving the seventh lifting rack to lift along the seventh guide rail, and the series-connected conveyor belt is disposed on an upper end surface of the seventh guide rail.

17. The sample cryogenic storage system according to claim 1, **characterized in that** the operation chamber further comprises an incoming identification member and a rotary cleaning member, wherein the incoming identification member is capable of identifying a barcode of the sample or the plate rack, and the rotary cleaning member is capable of cleaning the sample or the plate rack.
